(19) Europäisches Patentamt
European Patent Office
Office européen des brevets

(11) EP 1 178 951 B1

(12) EUROPÄISCHE PATENTSCHRIFT

(45) Veröffentlichungstag und Bekanntmachung des Hinweises auf die Patenterteilung:
**16.04.2003 Patentblatt 2003/16**

(21) Anmeldenummer: **00935014.1**

(22) Anmeldetag: **12.05.2000**

(51) Int Cl.[7]: **C07C 45/50**, C07F 17/00, C07F 17/02, B01J 31/20, B01J 31/18, B01J 31/22, B01J 31/24

(86) Internationale Anmeldenummer:
**PCT/EP00/04322**

(87) Internationale Veröffentlichungsnummer:
**WO 00/069801 (23.11.2000 Gazette 2000/47)**

(54) **ETA 5-PHOSPHOLYLKOMPLEXE UND IHRE VERWENDUNG IN DER HYDROFORMYLIERUNG**

ETA 5-PHOSPHOLYL COMPLEXES AND THEIR USE IN HYDROFORMYLATION

COMPLEXES ETA-5-PHOSPHOLYLE ET LEUR UTILISATION DANS LE CADRE DE L'HYDROFORMYLATION

(84) Benannte Vertragsstaaten:
**DE**

(30) Priorität: **12.05.1999 DE 19921730**

(43) Veröffentlichungstag der Anmeldung:
**13.02.2002 Patentblatt 2002/07**

(73) Patentinhaber: **BASF AKTIENGESELLSCHAFT**
**67056 Ludwigshafen (DE)**

(72) Erfinder:
- **AHLERS, Wolfgang**
  **D-67549 Worms (DE)**
- **MACKEWITZ, Thomas**
  **D-68219 Mannheim (DE)**
- **RÖPER, Michael**
  **D-67157 Wachenheim (DE)**
- **MATHEY, François**
  **F-91128 Palaiseau Cedex (FR)**
- **GANTER, Christian**
  **D-52056 Aachen (DE)**
- **BREIT, Bernhard**
  **D-69198 Schriesheim (DE)**

(74) Vertreter: **Isenbruck, Günter, Dr. et al**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
**DE-A- 19 621 967** **GB-A- 1 110 549**

- **MATHEY, FRANCOIS ET AL: "Etude de nouveaux catalyseurs d' hydroformylation des oléfines à base de complexes cobalt-carbonyle-phosphines" INF. CHIM. (1978), 179, 191-6 , XP002147313**
- **DATABASE CAPLUS [Online] CHEMICAL ABSTRACTS SERVICE, COLUMBUS, OHIO, US; AN: 1997:157731, MULLER, C. ET AL: "Phospha - ferrocenes as ligands: Syntheses, structures, coordination chemistry, and electrochemistry." retrieved from STN XP002147399 & BOOK OF ABSTRACTS, 213TH ACS NATIONAL MEETING, SAN FRANCISCO, APRIL 13-17 (1997), INOR-268 PUBLISHER: AMERICAN CHEMICAL SOCIETY, WASHINGTON, D. C. ,**
- **CHEMICAL ABSTRACTS, vol. 119, no. 18, 1. November 1993 (1993-11-01) Columbus, Ohio, US; abstract no. 194354, MUELLER, CHRISTIAN ET AL: "Triphosphaferrocenes as ligands: crystal and molecular structures of [Fe(.eta.5-C5Me5)(.eta.5-C2Bu-tert2P3)Cr(C O)5] and of the novel nickel complex [Fe(.eta.5-C5Me5)(.eta.5-C2Bu-tert2P3)Ni(C O)2]2 containing two interlinked [Fe(.eta.5-C5Me5)(.eta.5-C2Bu-tert2P3)] systems" XP002147423 & POLYHEDRON (1993), 12(11), 1383-90 ,**
- **Dillon, K.B.; Mathey, F.; Nixon, J.F. "Phosphorous: The Carbon Copy", J. Wiley, West Sussex; ISBN 0-471-97360-2; Chapter 9, Abschnitte 9.4: " 5-Phospholyl complexes" bis 9.6: " 5-Pentaphospholyl transition metal complexes", Seiten 294-342, XP000996501**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- Ganter, C.; Glinsböckel, C.; Ganter B. "New P,N-Chelate Ligands Based on Pyridyl-Substituted Phosphaferrocenes", European Journal of Inorganic Chemistry (1998), Seiten 1163-1168, XP000996198

## Beschreibung

Die vorliegende Erfindung betrifft die Verwendung von $\eta^5$-Phospholylkomplexen in Komplexen von Übergangsmetallen der VIII. Nebengruppe des Periodischen Systems der Elemente bei der Herstellung von Aldehyden durch Hydroformylierung von Olefinen mit $CO/H_2$ bei Temperaturen bis 200°C und Drücken bis 700 bar.

Jährlich werden weltweit etwa 7 Mio. Tonnen chemischer Produkte mit Hilfe der Hydroformylierung von Olefinen hergestellt. Es besteht deshalb ein großes wirtschaftliches Interesse daran, die Reaktion mit maximaler Selektivität und Aktivität zu betreiben.

Rhodiumhaltige phosphanmodifizierte Katalysatoren haben insbesondere für die Hydroformylierung von niedersiedenden Olefinen eine überragende Bedeutung gewonnen.

Zur Diskussion technischer Neuerungen wird auf M. Beller, B. Cornils, C. D. Frohning, C. W. Kohlpaintner, J. Mol. Catal. **1995,** 104, 17 verwiesen. Obwohl $\alpha$-Olefine sehr gut mit rhodiumhaitigen phosphanmodifizierten Katalysatoren hydroformylierbar sind, ist dieses Katalysatorsystem für interne und interne, verzweigte Olefine sowie für Olefine mit mehr als 7 Kohlenstoffatomen wenig geeignet. So werden interne Kohlenstoff-Kohlenstoff-Doppelbindungen nur sehr langsam in Gegenwart eines derartigen Katalysators hydroformyliert.

Rhodiumhaltige phosphitmodizifierte Katalysatoren werden neuerdings für die Hydroformylierung von niedersiedenden Olefinen vorgeschlagen (siehe M. Beller, B. Cornils, C. D. Frohning, C. W. Kohlpaintner, J. Mol. Catal **1995**, 104, 17). Sowohl $\alpha$-Olefine als auch kurzkettige interne Olefine wie 2-Buten oder 3-Pentensäuremethylester können sehr gut mit rhodiumhaltigen chelatphosphitmodifizierten Katalysatoren hydroformyliert werden. Allerdings ist dieses Katalysatorsystem für langkettige interne Olefine mit mehr als 7 Kohlenstoffatomen und interne, verzweigte Olefine wenig geeignet. Für diese Olefine haben sich einzähnige, sterisch gehinderte Monophosphite experimentell bewährt. Allerdings weisen Phosphite generell den Nachteil der Hydrolyseempfindlichkeit und der Tendenz zu Abbaureaktionen auf, wodurch deren technischer Einsatz eingeschränkt wird.

Der vorliegenden Erfindung lag daher die Aufgabe zugrunde, neue, gegen Hydrolyse und Abbaureaktionen unempfindliche Liganden, welche als Cokatalysatoren für die durch Übergangsmetalle der VIII. Nebengruppe, insbesondere Rhodium, katalysierte Hydroformylierung von Olefinen besonders geeignet sind, sowie ein Verfahren zur Herstellung von Aldehyden aus $\alpha$-Olefinen sowie aus internen und/oder verzweigten Olefinen oder aus höhersiedenden Olefinen (mit mehr als 7 Kohlenstoffatomen) bereitzustellen.

Obwohl viele phosphorhaltige Liganden für die Rhodium-Niederdruck-Hydroformylierung. bekannt sind (u.a. Arylphosphane, Alkylphosphane, Chelatphosphane, Monophosphite, Chelatphosphite, Phosphole und Phosphabenzole, siehe WO 97/46 507), wurde der Einsatz von $\eta^5$-Phospholyl- komplexen bisher nicht erwogen. Es wurde nun gefunden, daß diese Liganden als Cokatalysatoren für die Hydroformylierung von Olefinen hochaktiv und damit hervorragend geeignet sind. Bislang waren keine Anwendungen in der Hydroformylierung bekannt.

Die Aufgabe wird somit gelöst durch ein Verfahren zur Herstellung von Aldehyden durch Hydroformylierung von Olefinen mit $CO/H_2$ in Gegenwart von Komplexen von Übergangsmetallen der VIII. Nebengruppe des Periodensystems der Elemente bei Temperaturen von 20 bis 200°C und Drücken von Atmosphärendruck bis 700 bar, wobei man Katalysatoren mit zur Komplexbildung befähigten $\eta^5$-Phospholylkomplexen als Liganden verwendet. Dabei ist das Übergangsmetall ($\eta^1$-Metall) Rhodium.

Die $\eta^5$-Phospholylkomplexe sind ausgewählt aus den allgemeinen Strukturen I und V

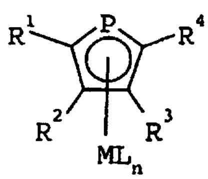

I

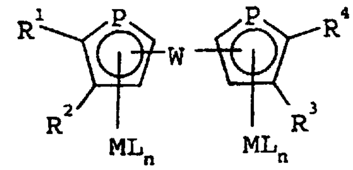

V

in denen $R^1$ bis $R^4$ unabhängig voneinander Wasserstoff, $C_{1-12}$-Alkyl-, $C_{7-12}$-Aralkyl-, $C_{7-12}$-Alkaryl- oder $C_{6-12}$-Arylreste bedeuten oder für die Reste $COO^-M^+$, $SO_3^-M^+$, $NR_3^+X^-$, OR, $NR_2$, COOR, SR, $(CHRCH_2O)_xR$, $(CH_2NR)_xR$ oder $(CH_2CH_2NR)_xR$ stehen, wobei R jeweils gleiche oder verschiedene Reste ausgewählt aus Wasserstoff, $C_{1-12}$-Alkyl- und $C_{6-12}$-Arylresten, $M^+$ ein Kation und $X^-$ ein Anion und x die Zahlen von 1 bis 120 bedeuten und wobei die Reste $R^1$ bis $R^4$ zu anellierten Ringen verbunden sein können, und in denen $ML_n$ für ein Metallkomplexfragment steht und W eine Brücke in Form einer kovalenten Bindung oder einer Kette aus 1 bis 10 Atomen, die Bestandteil einer cyclischen oder aromatischen Verbindung sein kann, oder eine Komplexverbindung ist und wobei die $\eta^5$-Phospholylkomplexeinheiten in 2-oder 3-Stellung zum Phosphor verknüpft sind, wobei die nicht für die Brücke verwendeten 2- und 3-Positionen durch Reste wie für $R^1$ bis $R^4$ beschrieben substituiert sein können, und wobei einer oder mehrere der Substituenten $R^1$ bis $R^4$ ein über eine weitere Brücke gebundener $\eta^5$-Phospholylkomplex der Formel I sein kann. Vorzugsweise ist W eine Brücke in Form einer Ferroceneinheit. Bevorzugte Reste $R^1$ bis $R^4$ sind H, Methyl und Phenyl.

L ist ausgewählt aus Cyclopentadienyl, CO, Halogen und Phosphan und Gemischen davon.

Als $\eta^5$-gebundene Metalle werden Zirconium, Wolfram, Mangan, Eisen, Ruthenium, Cobalt verwendet. Mindestens einer der Substituenten $R^1$ bis $R^4$ kann eine zusätzliche, zur Koordination befähigte dreibindige Phosphorgruppierung aufweisen, wodurch ein zwei- oder mehrzähniger Ligand entsteht. Besonders bevorzugt sind Phosphan-, Phosphinit-, Phosphonit- oder Phosphabenzolgruppierungen. Auch eine Stickstoffgruppierung, vorzugsweise Pyridingruppierung, kann vorliegen.

Beispielhaft werden folgende $\eta^5$-Phospholylkomplexe genannt:

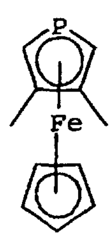

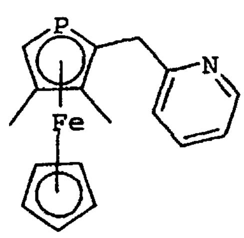

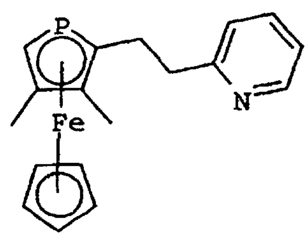

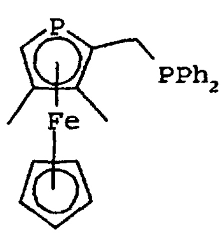

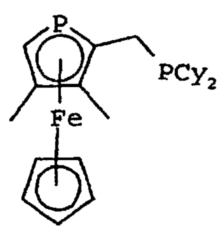

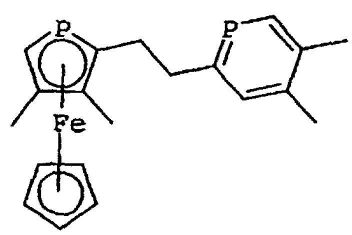

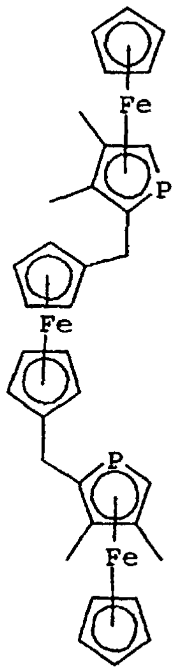

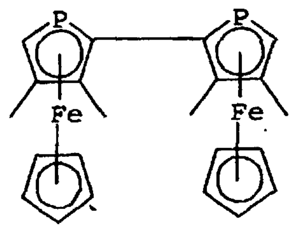

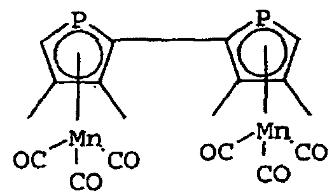

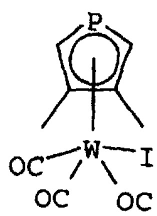

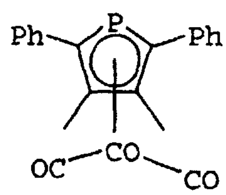

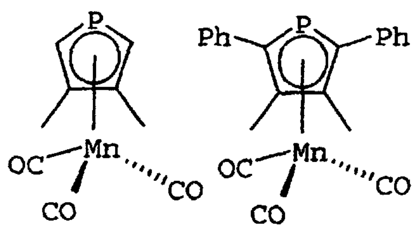

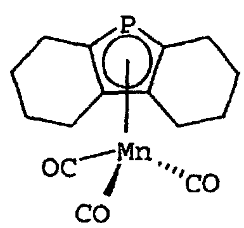

Diese Auflistung dient der Erläuterung der angewandten Begriffe. Cy bedeutet Cyclohexyl, Ph Phenyl.

Die Synthesen von $\eta^5$-Phospholyl- und $\eta^5$-Polyphospholylkomplexen sind z.B. in K. B. Dillon, F. Mathey, J. F. Nixon, Phosphorus: The Carbon Copy, J. Wiley, West Sussex, **1998**, Chapter 9 (und dort zitierte Literatur) sowie in C. Ganter, C. Glinsböckel, B. Ganter, Eur. J. Inorg. Chem. **1998,** 1163, in L. Brassat, B. Ganter, C. Ganter, Chem. Eur. J. **1998,** 4, 2148 und in C. Ganter, L. Brassat, B. Ganter, Chem. Ber./Recueil **1997,** 130, 1771 beschrieben.

WO 98/50392 ("Process to Prepare Bridged Phosphole-Cyclopentadienyl Compounds") beschreibt die Synthese von Phospholen und Phospholylkomplexen, sowie deren Verwendung als Polymerisationskatalysatoren.

Die bevorzugten wirksamen neuen Katalysatoren sind Komplexe der allgemeinen Formel (VI)

$$M'L'_{n'}(CO)_{m'} \qquad (VI)$$

mit der Bedeutung

M' Rhodium

L' zur Komplexbildung befähigter ein- oder mehrzähniger $\eta^5$-Phospholyl- komplex-Ligand, wie vorstehend definiert, oder Gemisch davon,

n' mindestens 1,

m' mindestens 1.

n' und m' können pro Äquivalent M' beispielsweise die Zahlen 1 bis 3 und die Summe von n'+m' kann 2 bis 6 bedeuten, und es können noch weitere Reste wie Hydrido oder Alkyl- oder Acylreste als Liganden enthalten sein.

Der Katalysator wird vorzugsweise zur Hydroformylierung von Olefinen eingesetzt. Der aktive Carbonylkomplex wird dabei in der Regel in situ, d.h. im Hydroformylierungsreaktor, aus einem Salz oder einer Verbindung des Metalls M', dem Liganden und Kohlenmonoxid gebildet; er kann aber auch getrennt hergestellt und eingesetzt werden.

Werden die Komplexkatalysatoren in situ erzeugt, setzt man einfache Precursorkomplexe wie Rhodiumdicarbonylacetylacetonat oder Rhodiumacetat in Gegenwart der entsprechenden Liganden den Reaktionsbedingungen aus, oder man versetzt Precursorkomplexe mit aktivierenden Zusätzen wie Brönsted- oder Lewis-Säuren oder Lewis-Basen.

Zur in situ-Bildung des Katalysators im Reaktionsgemisch setzt man den Liganden im molaren Verhältnis (gerechnet als Äquivalent Phosphor) zu Rhodium von vorzugsweise 1:1 bis 1000:1 ein. Bevorzugt wird dabei ein inertes Lösungsmittel verwendet. Besonders bevorzugte Lösungsmittel sind die Aldehyde, die durch Umsetzung des jeweiligen Olefins entstehen, sowie die syntheseeigenen Hochsieder, die durch Folgereaktionen des jeweiligen Aldehyds im Hydroformylierungsverfahren entstehen. Bei durch geeignete Substituenten hydrophilisierten Liganden werden bevorzugt Wasser, Alkohole oder andere Lösungsmittel eingesetzt.

Die Zusammensetzung des im erfindungsgemäßen Hydroformylierungsverfahren eingesetzten Synthesegases $CO/H_2$ kann in weiten Bereichen variiert werden. Beispielsweise kann Synthesegas mit $CO/H_2$-Molverhältnissen von 5:95 bis 70:30 erfolgreich eingesetzt werden, bevorzugt wird Synthesegas mit $CO/H_2$-Verhältnissen von 40:60 bis 60:40, besonders bevorzugt wird ein $CO/H_2$-Verhältnis von etwa 1:1 angewandt.

Hydroformylierungsreaktionen mit den oben beschriebenen Katalysatoren werden vorzugsweise bei einer Temperatur von 20 bis 200°C, insbesondere bei 50 bis 150°C, durchgeführt. Für jedes Katalysatorsystem kann zweckmäßig eine optimale Temperatur experimentell ermittelt werden. Der Reaktionsdruck kann je nach Cokatalysator und Substrat in einem Bereich von Normaldruck bis 700 bar, vorzugsweise bis 300 bar, liegen, wobei man normalerweise Reaktionen in einem Bereich bis zu etwa 30 bar als Niederdruck- und in einem Bereich bis zu etwa 100 bar als Mitteldruck- und über 100 bar als Hochdruckreaktionen bezeichnet.

Dabei arbeitet man in der Regel mit dem homogen im Reaktionsmedium gelösten Katalysator, der vom Austrag der Hydroformylierungsreaktion abgetrennt und in die Hydroformylierungsstufe zurückgeführt wird.

Man erhält in der Regel nahezu ausschließlich die entsprechenden Aldehyde in ausgezeichneten Ausbeuten. Besonders aktiv sind mit zweizähnigen Bis($\eta^5$phospholyl)komplexen der allgemeinen Formel V modifizierte Hydroformylierungskatalysatoren, mit welchen bei niedrigerem Synthesegasdruck die Hydroformylierung von Olefinen durchgeführt werden kann.

Das Katalysatorsystem ist rückführbar. Die Reaktionsausträge nach Rückführung zeigen gemäß $^{31}$P-NMR-Spektroskopie im Rahmen der Meßgenauigkeit keine oder nur eine geringe Zersetzung des Cokatalysators an.

Als erfindungsgemäß zu hydroformylierende Olefine kommen α-Olefine oder interne Olefine oder interne, verzweigte Olefine in Betracht. Auch funktionelle Gruppen werden toleriert. Beispielhaft werden folgende Olefine genannt: Ethylen, Propen, 1-Buten, 1-Octen, $C_{5-20}$ α-Olefine, lineare $C_{5-20}$ interne Olefine, 2-Buten, verzweigte interne Octen-Gemische, verzweigte interne Nonen-Gemische, verzweigte interne Dodecen-Gemische, Cyclohexen, Styrol, 3-Pentennitril, 4-Pentennitril, 3-Pentensäurealkylester, 4-Pentensäurealkylester, Polypropen, Polyisobutylen. Ebenfalls geeignete Substrate sind Di- oder Polyene mit isolierten oder konjugierten Doppelbindungen. Beispiele sind 1,3-Butadien, 1,5-Hexadien, Vinylcyclohexen, Dicylopentadien, 1,5,9-Cyclooctatrien, Butadienhomo- und -copolymere sowie Polyisobuten.

Die Erfindung wird durch die folgenden Beispiele näher erläutert:

**Beispiele**

Allgemeine Versuchsbeschreibung

Variante A: Rhodiumprecursor, Ligand und Lösungsmittel wurden unter Stickstoff-Inertgas in einem Schlenkrohr gemischt. Die erhaltene Lösung wurde in einen mit $CO/H_2$ (1:1) gespülten 70 ml oder 100 ml fassenden Autoklaven (Material HC) überführt. Es wurden 5 bar $CO/H_2$ (1:1) kalt aufgepresst. Unter kräftigem Rühren mit einem Begasungsrührer wurde das Reaktionsgemisch innerhalb von 30 min auf die gewünschte Temperatur erhitzt. Über eine Schleuse wurde dann das eingesetzte Olefin mit $CO/H_2$-Überdruck in den Autoklaven gepresst. Darauf wurde sofort mittels $CO/H_2$ (1:1) der gewünschte Reaktionsdruck eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit internem Standard und Korrekturfaktor durchgeführt.

Variante B: Rhodiumprecursor, Ligand und Lösungsmittel wurden unter Stickstoff-Inertgas in einem Schlenkrohr gemischt. Die erhaltene Lösung wurde in einen mit $CO/H_2$ (1:1) gespülten 70 ml oder 100 ml fassenden Autoklaven (Material HC) überfuhrt. Es wurden 5 bar $CO/H_2$ (1:1) kalt aufgepresst. Unter kräftigem Rühren mit einem Begasungsrührer wurde das Reaktionsgemisch innerhalb von 30 min auf 100°C erhitzt. Nach der Katalysatorpräformierung wurde der Autoklav abgekühlt und entspannt. Über eine Schleuse wurde dann das eingesetzte Olefin mit $CO/H_2$-Überdruck in den Autoklaven gepresst. Das Reaktionsgemisch wurde innerhalb von 30 min erneut auf die gewünschte Temperatur erhitzt. Darauf wurde sofort mittels $CO/H_2$ (1:1) der gewünschte Reaktionsdruck eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit internem Standard und Korrekturfaktor durchgeführt.

Beispiel A

Niederdruck-Hydroformylierung von 1-Octen

Ausgehend von 1,9 mg (0,007 mmol) Rhodiumdicarbonylacetylacetonat, 60,3 mg (0,179 mmol) 2-[2-(3,4-Dimethylphosphaferrocen-2-yl)ethyl]pyridin, 6,1 g (54 mmol) 1-Octen und 6,0 g Texanol® erhielt man bei 90°C, 10 bar $CO/H_2$ und 4 h gemäß der allgemeinen Versuchsdurchführung (Variante A, 100 ml. Autoklav) einen 1-Octen-Umsatz von 84 %. Die Ausbeute an Nonanalen betrug 45 %, die Selektivität zu n-Nonanal (n-Anteil) betrug 74 % und die Selektivität zu n-Nonanal und 2-Methyloctanal ($\alpha$-Anteil) betrug 100 %.

Beispiel B

Mitteldruck-Hydroformylierung von trans-2-Buten

Ausgehend von 1,8 mg (0,007 mmol) Rhodiumdicarbonylacetylaceton, 66,6 mg (0,198 mmol) 2-[2-(3,4-Dimethylphosphaferrocen-2-yl)ethyl]pyridin, 6,6 g (118 mmol) trans-2-Buten und 6,0 g Toluol erhielt man bei 100°C, 20 bar Gesamtdruck (Eigendruck des Olefins und $CO/H_2$) und 4 h gemäß der allgemeinen Versuchsdurchführung (Variante B, 100 ml Autoklav) einen trans-2-Buten-Umsatz von 23 %. Die Ausbeute an Aldehyden betrug 23 % und die Selektivität zu n-Pentanal (n-Anteil) betrug 29 %.

Beispiel 1

Niederdruck-Hydroformylierung von 1-Octen

Ausgehend von 1,9 mg (0,007 mmol) Rhodiumdicarbonylacetylacetonat, 36,5 mg (0,153 mmol) 3,4-Dimethylphosphaferrocen, 6,2 g (55 mmol) 1-Octen und 6,0 g Toluol erhielt man bei 90°C, 10 bar $CO/H_2$ und 4h gemäß der allgemeinen Versuchsdurchführung (Variante A, 100 ml Autoklav) einen 1-Octen-Umsatz von 100 %. Die Ausbeute an Nonanalen betrug 18 %, die Selektivität zu n-Nonanal (n-Anteil) betrug 75 % und die Selektivität zu n-Nonanal und 2-Methyloctanal ($\alpha$-Anteil) betrug 100 %.

Beispiel 2

Mitteldruck-Hydroformylierung von 1-Octen

Ausgehend von 2,3 mg (0,009 mmol) Rhodiumdicarbonylacetylacetonat, 38,5 mg (0,166 mmol) 3,4-Dimethylphosphaferrocen, 6,3 g (56 mmol) 1-Octen und 6,0 g Toluol erhielt man bei 90°C, 40 bar $CO/H_2$ und 4h gemäß der allgemeinen Versuchsdurchführung (Variante A, 100 ml Autoklav) einen 1-Octen-Umsatz von 99 %. Die Ausbeute an Nonanalen betrug 73 %, die Selektivität zu n-Nonanal (n-Anteil) betrug 63 % und die Selektivität zu n-Nonanal und 2-Methyloctanal ($\alpha$-Anteil) betrug 97 %.

Beispiel 3

Mitteldruck-Hydroformylierung von trans-2-Buten

Ausgehend von 4,4 mg (0,017 mmol) Rhodiumdicarbonylacetylacetonat, 96,2 mg (0,415 mmol) 3,4-Dimethylphosphaferrocen, 14,5 g (258 mmol) trans-2-Buten und 15,0 g Toluol erhielt man bei 100°C, 60 bar Gesamtdruck (Eigendruck des Olefins und $CO/H_2$) und 4 h gemäß der allgemeinen Versuchsdurchführung (Variante B, 100 ml Autoklav) einen trans-2-Buten-Umsatz von 73 %. Die Ausbeute an Aldehyden betrug 73 % und die Selektivität zu n-Pentanal (n-Anteil) betrug 17%.

Beispiel 4

Mitteldruck-Hydroformylierung von Octen-N2

Ausgehend von 4,6 mg (0,018 mmol) Rhodiumdicarbonylacetylacetonat, 94,9 mg (0,409 mmol) 3,4-Dimethylphosphaferrocen, 15,7 g (140 mmol) Octen-N2 (Isooctengemisch, Verzweigungsgrad 1,06) und 15,0 g Texanol® erhielt man bei 100°C, 60 bar Gesamtdruck gemäß der allgemeinen Versuchsdurchführung (Variante A, 100 ml Autoklav) einen Octen-N2-Umsatz von 32 % nach 4 h Reaktionszeit und 55 % nach 24 h Reaktionszeit. Die Ausbeute an Nonanalen betrug 32 % nach 4 h Reaktionszeit und 55 % nach 24 h Reaktionszeit.

Beispiel 5

Mitteldruck-Hydroformylierung von 1-Octen

Ausgehend von 2,0 mg (0,008 mmol) Rhodiumdicarbonylacetylacetonat, 53,0 mg (0,164 mmol) 2-[(3,4-Dimethylphosphaferrocen-2-yl)methyl]pyridin, 6,8 mg (61 mmol) 1-Octen und 6,0 g Texanol® erhielt man bei 90°C, 40 bar $CO/H_2$ und 4 h gemäß der allgemeinen Versuchsdurchführung (Variante A, 100 ml Autoklav) einen 1-Octen-Umsatz von 99 %. Die Ausbeute an Nonanalen betrug 82 %, die Selektivität zu n-Nonanal (n-Anteil) betrug 65 %, und die Selektivität zu n-Nonanal und 2-Methyloctanal ($\alpha$-Anteil) betrug 97 %.

Beispiel 6

Mitteldruck-Hydroformylierung von 1-Octen (Rückführung)

Die Aldehyde im Reaktionsgemisch von Beispiel 5 wurden bei 100°C unter Vakuum abdestilliert. Zurück blieb eine homogene Lösung, bestehend aus Aktivkatalysator, Texanol® und reaktionseigenen Hochsiedern. Hierzu wurden unter Luftausschluß 6,8 g (61 mmol) 1-Octen gegeben. Die erhaltene Lösung wurde in einen mit $CO/H_2$ (1:1) gespülten 100 ml Autoklav (Material HC) überführt. Es wurden 5 bar $CO/H_2$ (1:1) kalt aufgepresst. Unter käftigem Rühren mit einem Begasungsrührer wurde das Reaktionsgemisch innerhalb von 30 min auf 90°C erhitzt. Daraufhin wurde sofort mittels $CO/H_2$ (1:1) ein Reaktionsdruck von 40 bar $CO/H_2$ eingestellt. Während der Reaktion wurde der Druck im Reaktor durch Nachpressen über einen Druckregler auf Druckniveau gehalten. Nach der Reaktionszeit von 4 h wurde der Autoklav abgekühlt, entspannt und entleert. Eine Analyse des Reaktionsgemisches wurde mittels GC mit internem Standard und Korrekturfaktor durchgeführt. Der 1-Octen-Umsatz betrug 67 %, die Ausbeute an Nonanalen betrug 57 %, die Selektivität zu n-Nonanal (n-Anteil) betrug 73 %, und die Selektivität zu n-Nonanal und 2-Methyloctanal ($\alpha$-Anteil) betrug 100 %. Ein $^{31}P$-NMR-Spektrum des Reaktionsaustrages zeigt im Rahmen der Meßgenauigkeit das unveränderte Vorliegen des Cokatalysators.

Beispiel 7

Niederdruck-Hydroformylierung von 1-Octen

Ausgehend von 2,1 mg (0,008 mmol) Rhodiumdicarbonylacetylacetonat, 57,8 mg (0,171 mmol) 2-[2-(3,4-Dimethylphosphaferrocen-2-yl)ethyl]pyridin, 6,3 mg (56 mmol) 1-Octen und 6,0 g Texanol® erhielt man bei 90°C, 20 bar $CO/H_2$ und 4 h gemäß der allgemeinen Versuchsdurchführung (Variante A, 70 ml Autoklav) einen 1-Octen-Umsatz von 99 %. Die Ausbeute an Nonanalen betrug 76 %, die Selektivität zu n-Nonanal (n-Anteil) betrug 67 %, und die Selektivität zu n-Nonanal und 2-Methyloctanal ($\alpha$-Anteil) betrug 97 %.

Beispiel 8

Mitteldruck-Hydroformylierung von Isobuten

Ausgehend von 3,4 mg (0,011 mmol) Rhodiumdicarbonylacetylacetonat, 104,0 mg (0,322 mmol) 2-[2-(3,4-Dimethylphosphaferrocen-2-yl)ethyl]pyridin, 12,3 g (219 mmol) Isobuten und 10,0 g Texanol® erhielt man bei 100°C, 40 bar Gesamtdruck (Eigendruck des Olefins und $CO/H_2$) und 4 h gemäß der allgemeinen Versuchsdurchführung (Variante B, 70 ml Autoklav) einen Isobuten-Umsatz von 39 %. Die Ausbeute an Aldehyden betrug 39 %, und die Selektivität zu Isovaleraldehyd betrug 100 %.

Beispiel 9

Niederdruck-Hydroformylierung von 1-Octen

Ausgehend von 1,9 mg (0,007 mmol) Rhodiumdicarbonylacetylacetonat, 61,5 mg (0,164 mmol) 2,5-Diphenylphosphacymantren, 7,3 mg (65 mmol) 1-Octen und 6,0 g Texanol® erhielt man bei 90°C, 10 bar $CO/H_2$ und 4 h gemäß der allgemeinen Versuchsdurchführung (Variante A, jedoch Präformierung bei 90°C und 2 bar $CO/H_2$, 70 ml Autoklav) einen 1-Octen-Umsatz von 99 %. Die Ausbeute an Nonanalen betrug 15 %, die Selektivität zu n-Nonanal (n-Anteil) betrug 47 %, und die Selektivität zu n-Nonanal und 2-Methyloctanal (α-Anteil) betrug 85 %.

Beispiel 10

Niederdruck-Hydroformylierung von 1-Octen (Rückführung)

Die Aldehyde im Reaktionsgemisch von Beispiel 9 wurden bei 100°C unter Vakuum abdestilliert. Zurück blieb eine homogene Lösung bestehend aus Aktivkatalysator, Texanol® und reaktionseigenen Hochsiedern. Hierzu wurden unter Luftausschluß 6,7 g (60 mmol) 1-Octen gegeben. Die erhaltene Lösung wurde in einen mit $CO/H_2$ (1:1) gespülten 70 ml Autoklav (Material HC) überführt. Es wurden 2 bar $CO/H_2$ (1:1) kalt aufgepresst. Unter kräftigem Rühren mit einem Begasungsrührer wurde das Reaktionsgemisch innerhalb von 30 min auf 90°C erhitzt. Daraufhin wurde sofort mittels $CO/H_2$ (1:1) der gewünschte Reaktionsdruck eingestellt. Während der Reaktion wurde der Druck im Reaktor durch Nachpressen über einen Druckregler auf Druckniveau gehalten. Nach der Reaktionszeit von 4 h wurde der Autoklav abgekühlt, entspannt und entleert. Eine Analyse des Reaktionsgemisches wurde mittels GC mit internem Standard und Korrekturfaktor durchgeführt. Der 1-Octen-Umsatz betrug 99 %, die Ausbeute an Nonanalen betrug 32 %, die Selektivität zu n-Nonanal (n-Anteil) betrug 55 %, und die Selektivität zu n-Nonanal und 2-Methyloctanal (α-Anteil) betrug 90 %.

Beispiel 11

Mitteldruck-Hydroformylierung von 1-Octen

Ausgehend von 2,1 mg (0,008 mmol) Rhodiumdicarbonylacetylacetonat, 70,7 mg (0,189 mmol) 2,5-Diphenylphosphacymantren, 6,0 g (23 mmol) 1-Octen und 6,0 g Texanol® erhielt man bei 90°C, 40 bar $CO/H_2$ und 4 h gemäß der allgemeinen Versuchsdurchführung (Variante A, 70 ml Autoklav) einen 1-Octen-Umsatz von 100 %. Die Ausbeute an Nonanalen betrug 83 %, die Selektivität zu n-Nonanal (n-Anteil) betrug 40 %, und die Selektivität zu n-Nonanal und 2-Methyloctanal (α-Anteil) betrug 77 %.

Beispiel 12

Mitteldruck-Hydroformylierung von 1-Octen (Rückführung)

Die Aldehyde im Reaktionsgemisch von Beispiel 11 wurden bei 100°C unter Vakuum abdestilliert. Zurück blieb eine homogene Lösung, bestehend aus Aktivkatalysator, Texanol® und reaktionseigenen Hochsiedern. Hierzu wurden unter Luftausschluß 6,0 g (23 mmol) 1-Octen gegeben. Die erhaltene Lösung wurde in einen mit $CO/H_2$ (1:1) gespülten 70 ml Autoklav (Material HC) überführt. Es wurden 5 bar $CO/H_2$ (1:1) kalt aufgepresst. Unter kräftigem Rühren mit einem Begasungsrührer wurde das Reaktionsgemisch innerhalb von 30 min auf 90°C erhitzt. Daraufhin wurde sofort mittels $CO/H_2$ (1:1) ein Reaktionsdruck von 40 bar $CO/H_2$ eingestellt. Während der Reaktion wurde der Druck im Reaktor durch Nachpressen über einen Druckregler auf Druckniveau gehalten. Nach der Reaktionszeit von 4 h wurde der Autoklav abgekühlt, entspannt und entleert. Eine Analyse des Reaktionsgemisches wurde mittels GC

mit internem Standard und Korrekturfaktor durchgeführt. Der 1-Octen-Umsatz betrug 100 %, die Ausbeute an Nonanalen betrug 92 %, die Selektivität zu n-Nonanal (n-Anteil) betrug 26 % und die Selektivität zu n-Nonanal und 2-Methyloctanal (α-Anteil) betrug 65 %. Ein $^{31}P$-NMR-Spektrum des Reaktionaustrages zeigte im Rahmen der Meßgenauigkeit das unveränderte Vorliegen des Cokatalysators.

Beispiel 13

Mitteldruck-Hydroformylierung von trans-2-Buten

Ausgehend von 2,1 mg (0,008 mmol) Rhodiumdicarbonylacetylacetonat, 66,3 mg (0,177 mmol) 2,5-Diphenylphosphacymantren, 3,5 g (62 mmol) trans-2-Buten und 6,0 g Texanol® erhielt man bei 100°C, 40 bar Gesamtdruck (Eigendruck des Olefins und $CO/H_2$) und 4 h gemäß der allgemeinen Versuchsdurchführung (Variante B, 70 ml Autoklav) einen trans-2-Buten-Umsatz von 66 %. Die Ausbeute an Aldehyden betrug 66 %, und die Selektivität zu n-Pentanal (n-Anteil) betrug 37 %.

Beispiel 14

Mitteldruck-Hydroformylierung von trans-2-Buten (Rückführung)

Die Aldehyde im Reaktionsgemisch von Beispiel 13 wurden bei 70°C unter Vakuum abdestilliert. Zurück blieb eine homogene Lösung, bestehend aus Aktivkatalysator, Texanol® und reaktionseigenen Hochsiedern. Die Lösung wurde in einen mit $CO/H_2$ (1:1) gespülten 70 ml Autoklav (Material HC) überführt. Über eine Schleuse wurden dann 4,5 g (80 mmol) trans-2-Buten mit $CO/H_2$-Überdruck in den Autoklaven gepresst. Unter kräftigem Rühren mit einem Begasungsrührer wurde das Reaktionsgemisch innerhalb von 30 min auf 100°C erhitzt. Daraufhin wurde sofort mittels $CO/H_2$ (1:1) ein Reaktionsdruck von 40 bar (Eigendruck des Olefins und $CO/H_2$) eingestellt. Während der Reaktion wurde der Druck im Reaktor durch Nachpressen über einen Druckregler auf Druckniveau gehalten. Nach der Reaktionszeit von 4 h wurde der Autoklav abgekühlt, entspannt und entleert. Eine Analyse des Reaktionsgemisches wurde mittels GC mit internem Standard und Korrekturfaktor durchgeführt. Der trans-2-Buten-Umsatz betrug 65 %, die Ausbeute an Nonanalen betrug 65 %, und die Selektivität zu n-Pentanal (n-Anteil) betrug 35 %. Ein $^{31}P$-NMR-Spektrum des Reaktionsaustrages zeigte im Rahmen der Meßgenauigkeit das nahezu unveränderte Vorliegen des Cokatalysators.

Vergleichsbeispiel 1

Mitteldruck-Hydroformylierung von trans-2-Buten

Ausgehend von 4,9 mg (0,019 mmol) Rhodiumdicarbonylacetylacetonat, 113,1 mg (0,431 mmol) Triphenylphosphan, 12,9 g (230 mmol) trans-2-Buten und 15,3 g Toluol erhielt man bei 100°C, 40 bar Gesamtdruck (Eigendruck des Olefins und $CO/H_2$) und 4 h gemäß der allgemeinen Versuchsdurchführung (Variante B, 100 ml Autoklav) einen trans-2-Buten-Umsatz von 47 %. Die Ausbeute an Aldehyden betrug 47 %, und die Selektivität zu n-Pentanal (n-Anteil) betrug 3 %.

Beispiel 15

Mitteldruck-Hydroformylierung von Octen-N2

Ausgehend von 3,4 mg (0,013 mmol) Rhodiumdicarbonylacetylacetonat, 102,8 mg (0,275 mmol) 2,5-Diphenylphosphacymantren, 10,7 g (95 mmol), Octen-N2 (Isooctengemisch, Verzweigungsgrad 1,06) und 10,0 g Texanol® erhielt man bei 100°C, 60 bar Gesamtdruck gemäß der allgemeinen Versuchsdurchführung (Variante A, 100 ml Autoklav) einen Octen-N2-Umsatz von 74 % nach 24 h Reaktionszeit. Die Ausbeute an Nonanalen betrug 74 % nach 24 h Reaktionszeit.

Beispiel 16

Mitteldruck-Hydroformylierung von Octen-N2 (Rückführung)

Die Aldehyde im Reaktionsgemisch von Beispiel 15 wurden bei 100°C unter Vakuum abdestilliert. Zurück blieb eine homogene Lösung, bestehend aus Aktivkatalysator, Texanol® und reaktionseigenen Hochsiedern. Die Lösung wurde in einen mit $CO/H_2$ (1:1) gespülten 100 ml Autoklav (Material HC) überführt. Hierzu wurden unter Luftausschluß 10,0 g (89 mmol) Octen-N2 gegeben. Die erhaltene Lösung wurde in einen mit $CO/H_2$ (1:1) gespülten 100 ml Autoklav (Material HC) überführt. Es wurden 5 bar $CO/H_2$ (1:1) kalt aufgepresst. Unter kräftigem Rühren mit einem Begasungsrührer wurde das Reaktionsgemisch innerhalb von 30 min auf 90°C erhitzt. Daraufhin wurde sofort mittels $CO/H_2$ (1:1) ein Reaktionsdruck von 60 bar $CO/H_2$ eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit internem Standard und Korrekturfaktor durchgeführt. Der Octen-N2-Umsatz betrug 60 % nach 4 h Reaktionszeit und 88 % nach 24 h Reaktionszeit. Die Ausbeute an Nonanalen betrug 60 % nach 4 h Reaktionszeit und 88 % nach 24 h Reaktionszeit. Ein $^{31}P$-NMR-Spektrum des Reaktionsaustrages zeigte im Rahmen der Meßgenauigkeit das nahezu unveränderte Vorliegen des Cokatalysators.

Vergleichsbeispiel 2

Mitteldruck-Hydroformylierung von Octen-N2

Ausgehend von 4,4 mg (0,017 mmol) Rhodiumdicarbonylacetylacetonat, 107,3 mg (0,409 mmol) Triphenylphosphan, 16,2 g (144 mmol) Octen-N2 (Isooctengemisch, Verzweigungsgrad 1,06) und 15,3 g Texanol® erhielt man bei 100°C, 60 bar Gesamtdruck gemäß der allgemeinen Versuchsdurchführung (Variante A, 100 ml Autklav) einen Octen-N2-Umsatz von 39 % nach 24 h Reaktionszeit. Die Ausbeute an Nonanalen betrug 39 % nach 24 h Reaktionszeit.

Beispiel 17

Niederdruck-Hydroformylierung von 1-Octen

0,7 mg (0,003 mmol) Rhodiumdicarbonylacetylacetonat, 17,8 mg (0,026 mmol) 1,1'-Bis[(3,4-dimethylphosphaferrocen-2-yl)methyl]ferrocen und 2,3 ml Toluol wurden unter Stickstoff-Inertgas in einem Schlenkrohr gemischt. Die erhaltene Lösung wurde in einen mit $CO/H_2$ (1:1) gespülten 50 ml Glasautoklaven überführt. Es wurden 10 bar $CO/H_2$ (1:1) kalt aufgepresst. Unter kräftigem Rühren mit einem Begasungsrührer wurde das Reaktionsgemisch innerhalb von 30 min auf eine Temperatur von 100°C erhitzt. Danach wurde auf eine Temperatur von 80°C temperiert, und anschließend wurde der Autoklav entspannt. Mittels einer Spritze wurden 2,2 g (20 mmol) 1-Octen im $CO/H_2$-Gegenstrom zugegeben. Daraufhin wurde sofort mittels $CO/H_2$, (1:1) ein Reaktionsdruck von 10 bar eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit Korrekturfaktoren durchgeführt. Der 1-Octen-Umsatz betrug 92 %, die Ausbeute an Nonanalen betrug 83 %, die Selektivität zu n-Nonanal (n-Anteil) betrug 69 % und die Selektivität zu n-Nonanal und 2-Methyloctanal ($\alpha$-Anteil) betrug 100 %.

Beispiel 18

Niederdruck-Hydroformylierung von 1-Octen

0,7 mg (0,003 mmol) Rhodiumdicarbonylacetylacetonat, 8,8 mg (0,013 mmol) 1,1'-Bis[(3,4-dimethylphosphaferrocen-2-yl)methyl]ferrocen und 2,3 ml Toluol wurden unter Stickstoff-Inertgas in einem Schlenkrohr gemischt. Die erhaltene Lösung wurde in einen mit $CO/H_2$ (1:1) gespülten 50 ml Glasautoklaven überführt. Es wurden 10 bar $CO/H_2$ (1:1) kalt aufgepresst. Unter kräftigem Rühren mit einem Begasungsrührer wurde das Reaktionsgemisch innerhalb von 30 min auf eine Temperatur von 100°C erhitzt. Danach wurde auf eine Temperatur von 80°C temperiert, und anschließend wurde der Autoklav entspannt. Mittels einer Spritze wurden 2,2 g (20 mmol) 1-Octen im $CO/H_2$-Gegenstrom zugegeben. Daraufhin wurde sofort mittels $CO/H_2$ (1:1) ein Reaktionsdruck von 10 bar eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit Korrekturfaktoren durchgeführt. Der 1-Octen-Umsatz betrug 71 %, die Ausbeute an Nonanalen betrug 60 %, die Selektivität zu n-Nonanal (n-Anteil) betrug 70 %, und die Selektivität zu n-Nonanal und 2-Methyloctanal ($\alpha$-Anteil) betrug 100 %.

Beispiel 19

Mitteldruck-Hydroformylierung von trans-2-Buten

Ausgehend von 4,2 mg (0,016 mmol) Rhodiumdicarbonylacetylacetonat, 110,0 mg (0,163 mmol) 1,1'-Bis[(3,4-dimethylphosphaferrocen-2-yl)methyl]ferrocen, 14,7 g (262 mmol) trans-2-Buten und 15,0 g Toluol erhielt man bei 100°C, 40 bar Gesamtdruck (Eigendruck des Olefins und $CO/H_2$) und 4 h gemäß der allgemeinen Versuchsdurchführung (Variante B, jedoch Präformierung bei 80°C und 20 bar, 100 ml Autoklav) einen trans-2-Buten-Umsatz von 72 %. Die Ausbeute an Aldehyden betrug 72 % und die Selektivität zu n-Pentanal (n-Anteil) betrug 13 %.

Beispiel 20

Mitteldruck-Hydroformylierung von trans-2-Buten (Rückführung)

Die Aldehyde im Reaktionsgemisch von Beispiel 19 wurden bei 70°C unter Vakuum abdestilliert. Zurück blieb eine homogene Lösung, bestehend aus Aktivkatalysator, Toluol und reaktionseigenen Hochsiedern, welche mit Toluol auf eine Gesamtmenge von 15 g ergänzt wurde. Die erhaltene Lösung wurde in einen mit $CO/H_2$ (1:1) gespülten 100 ml Autoklav (Material HC) überfuhrt. Es wurden 20 bar $CO/H_2$ (1:1) kalt aufgepresst. Unter kräftigem Rühren mit einem Begasungsrührer wurde das Reaktionsgemisch innerhalb von 30 min auf 80°C erhitzt. Nach der Katalysatorpräformierung wurde der Autoklav abgekühlt und entspannt. Über eine Schleuse wurden dann 15,7 g (280 mmol) trans-2-Buten und $CO/H_2$-Überdruck in den Autoklaven gepresst. Das Reaktionsgemisch wurde innerhalb von 30 min erneut auf eine Temperatur von 100°C erhitzt. Daraufhin wurde sofort mittels $CO/H_2$ (1:1) ein Reaktionsdruck von 40 bar (Eigendruck des Olefins und $CO/H_2$) eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit Korrekturfaktoren durchgeführt. Der trans-2-Buten-Umsatz betrug 54 %, die Ausbeute an Pentanalen betrug 54 % und die Selektivität zu n-Pentanal (n-Anteil) betrug 21 %.

Beispiel 21

Hydroformylierung von 3-Pentennitril mit 1,1'-Bis[(3,4-dimethylphosphaferrocen-2-yl)methyl]ferrocen

0,7 mg (0,003 mmol) $Rh(CO)_2$acac wurden mit 3 g Xylol und 17,8 mg (0,026 mmol) Ligand vorgelegt und bei 100°C und 15 bar Synthesegas 30 min lang präformiert. Anschließend wurden 1,5 g (18,5 mmol) 3-Pentennitril zugespritzt und der Druck auf 15 bar eingestellt. Nach einer Reaktionszeit von 4 h wurde bei einem Umsatz von 71 % eine Aldehydausbeute von 69 % erzielt. Die Linearität betrug 2 %, der $\alpha$-Anteil 26 %.

Beispiel 22

Hydroformylierung von 4-Pentennitril mit 1,1'-Bis[(3,4-dimethylphosphaferrocen-2-yl)methyl]ferrocen

0,7 mg (0,003 mmol) $Rh(CO)_2$acac wurden mit 3 g Xylol und 17,8 mg (0,026 mmol) Ligand vorgelegt und bei 100°C und 15 bar Synthesegas 30 min lang präformiert. Anschließend wurden 1,5 g (18,5 mmol) 4-Pentennitril zugespritzt und der Druck auf 15 bar eingestellt. Nach einer Reaktionszeit von 4 h wurde bei einem Umsatz von 42 % eine Aldehydausbeute von 42 % erzielt. Die Linearität betrug 52 %, der $\alpha$-Anteil 100 %.

**Patentansprüche**

1. Verfahren zur Herstellung von Aldehyden durch Hydroformylierung von Olefinen mit $CO/H_2$ in Gegenwart von Komplexen von Übergangsmetallen der VIII. Nebengruppe des Periodensystems der Elemente bei Temperaturen von 20 bis 200°C und Drücken von Atmosphärendruck bis 700 bar, **dadurch gekennzeichnet, daß** man Katalysatoren mit zur Komplexbildung befähigten $\eta^5$-Phospholylkomplexen als Liganden verwendet, das als $\eta^1$-Metall eingesetzte Übergangsmetall Rhodium ist und die $\eta^5$-Phospholylkomplexe ausgewählt sind aus den allgemeinen Strukturen I und V

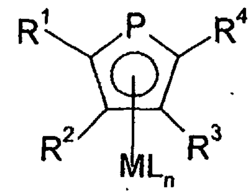

I

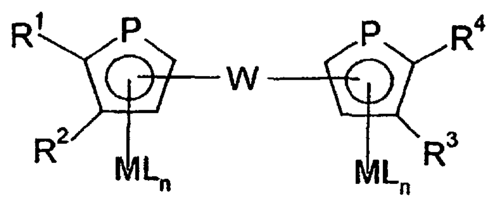

V

in denen $R^1$ bis $R^4$ unabhängig voneinander Wasserstoff, $C_{1-12}$-Alkyl-, $C_{7-12}$-Aralkyl-, $C_{7-12}$-Alkaryl- oder $C_{6-12}$-Arylreste bedeuten, wobei mindestens einer der Substituenten $R^1$ bis $R^4$ eine zusätzliche Stickstoffgruppierung oder zur Koordination befähigte dreibindige Phosphorgruppierung aufweisen kann, wodurch ein zwei- oder mehrzähniger Ligand entsteht, oder für die Reste $COO^-M^+$, $SO_3^-M^+$, $NR_3^+X^-$,OR, $NR_2$, COOR, SR, $(CHRCH_2O)_xR$, $(CH_2NR)_xR$ oder $(CH_2CH_2NR)_xR$ stehen, wobei R jeweils gleiche oder verschiedene Reste ausgewählt aus Wasserstoff, $C_{1-12}$-Alkyl- und $C_{6-12}$-Arylresten, $M^+$ ein Kation und $X^-$ ein Anion und x die Zahlen von 1 bis 120 bedeuten und wobei die Reste $R^1$ bis $R^4$ zu anellierten Ringen verbunden sein können, und in denen $ML_n$ für ein Metallkomplexfragment steht und W eine Brücke in Form einer kovalenten Bindung oder einer Kette aus 1 bis 10 Atomen, die Bestandteil einer cyclischen oder aromatischen Verbindung sein kann, oder einer Komplexverbindung ist und wobei die $\eta^5$-Phospholylkomplexeinheiten in 2-oder 3-Stellung zum Phosphor verknüpft sind, wobei die nicht für die Brücke verwendeten 2- und 3-Positionen durch Reste wie für $R^1$ bis $R^4$ beschrieben substituiert sein können, und wobei einer oder mehrere der Substituenten $R^1$ bis $R^4$ ein über eine weitere Brücke gebundener $\eta^5$-Phospholylkomplex der Formel I sein kann, wobei M ausgewählt ist aus Zr, W, Mn, Fe, Ru, Co und L ausgewählt ist aus Cyclopentadienyl, CO, Halogen und Phosphan und Gemischen davon.

**2.** Verfahren nach Anspruch 1, **dadurch gekennzeichnet, daß** die $\eta^5$-Phospholylkomplexe ausgewählt sind aus den folgenden Strukturen, in denen Ph Phenyl und Cy Cyclohexyl bedeuten,

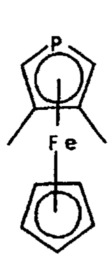

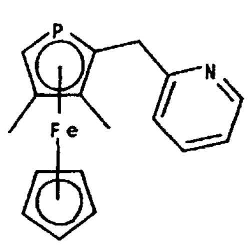

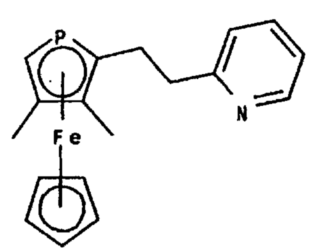

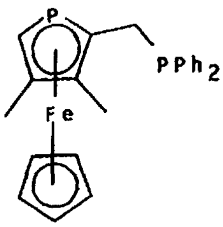 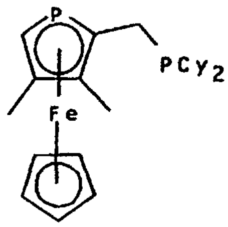 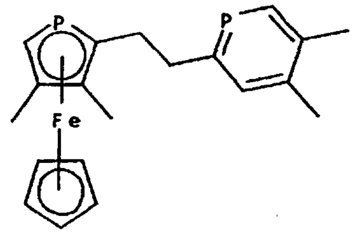

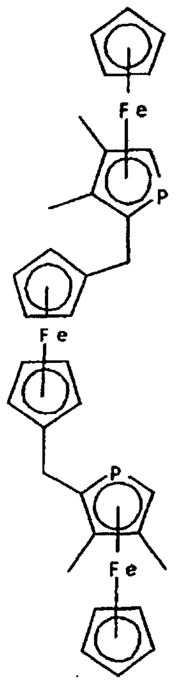 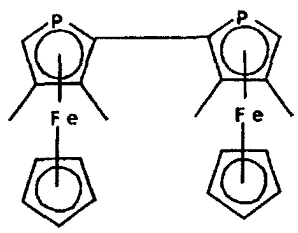 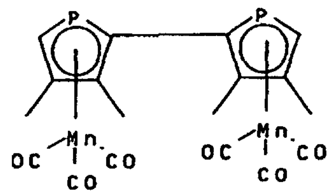

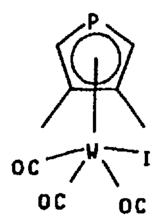 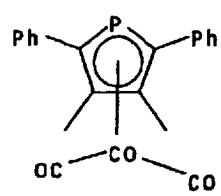

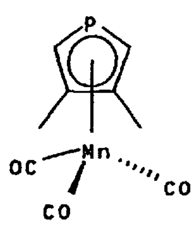 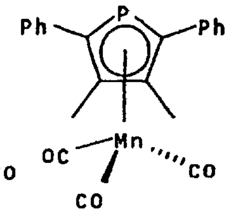 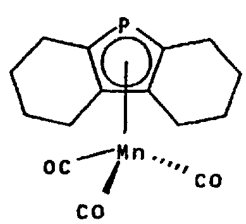

**3.** Komplexe der allgemeinen Formel (VI)

$$M'L'_{n'}(CO)_{m'} \quad (VI)$$

mit der Bedeutung

M' Rhodium

L' zur Komplexbildung befähigter ein- oder mehrzähniger $\eta^5$-Phospholylkomplex-Ligand, wie in Anspruch 1 oder 2 definiert, oder Gemisch davon,

n' mindestens 1,

m' mindestens 1.

**4.** Verwendung von Komplexen gemäß Anspruch 3 als Katalysator bei der Hydroformylierung von Olefinen.

## Claims

**1.** A process for preparing aldehydes by hydroformylation of olefins using $CO/H_2$ in the presence of complexes of transition metals of transition group VIII of the Periodic Table of the Elements at from 20 to 200°C and pressures from atmospheric pressure to 700 bar, wherein catalysts having $\eta^5$-phospholyl complexes capable of complex formation as ligands are used, the transition metal used as $\eta^1$-metal is rhodium and the $\eta^5$-phospholyl complexes are selected from among the structures I and V

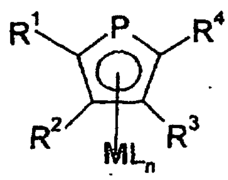

I

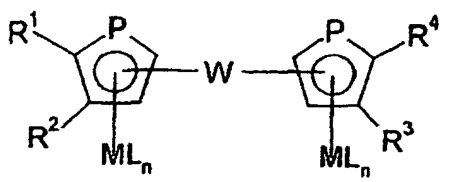

V

in which $R^1$ to $R^4$ are, independently of one another, hydrogen, $C_{1-12}$-alkyl, $C_{7-12}$-aralkyl, $C_{7-12}$-alkaryl or $C_{6-12}$-aryl radicals, where at least one of the substituents $R^1$ to $R^4$ can have an additional nitrogen group or an additional trivalent phosphorus group capable of coordination, resulting in a bidentate or polydentate ligand, or the radicals $COC^-M^+$, $SO_3^-M^+$, $NR_3^+X^-$, OR, $NR_2$, COOR, SR, $(CHRCH_2O)_xR$, $(CH_2NR)_xR$ or $(CH_2CH_2NR)_xR$, where R are in each case identical or different radicals selected from among hydrogen, $C_{1-12}$-alkyl and $C_{6-12}$-aryl radicals, $M^+$ is a cation and $X^-$ is an anion and x is from 1 to 120, where the radicals $R^1$ to $R^4$ may be joined to form fused rings, and in which $ML_n$ is a metal complex fragment and W is a bridge in the form of a covalent bond or a chain of from 1 to 10 atoms which may be part of a cyclic or aromatic compound, or is a complex, and where the $\eta^5$-phospholyl complex units are linked in the 2 or 3 position relative to the phosphorus, where the 2 and 3 positions not used for the bridge may be substituted by radicals as described for $R^1$ to $R^4$, and where one or more of the substituents

$R^1$ to $R^4$ may be a η5-phospholyl complex of the formula I bound via a further bridge, where M is selected from among Zr, W, Mn, Fe, Ru, Co, and L is selected from among cyclopentadienyl, CO, halogen and phosphine and mixtures thereof.

**2.** A process as claimed in claim 1, wherein the η5-phospholyl complexes are selected from among the following structures, in which Ph is phenyl and Cy is cyclohexyl,

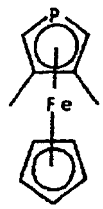
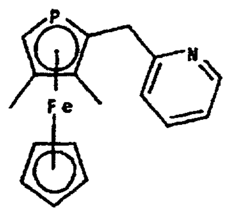
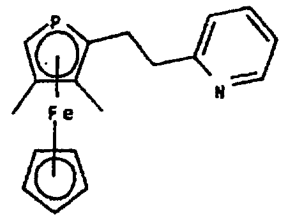

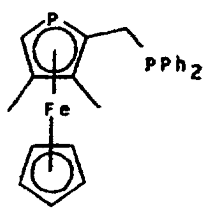
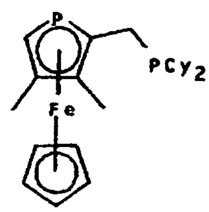
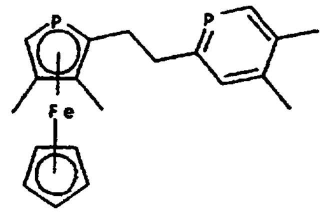

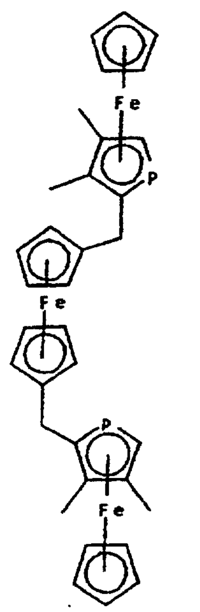
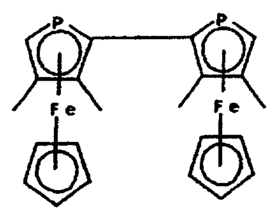
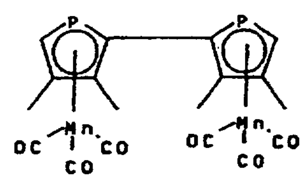

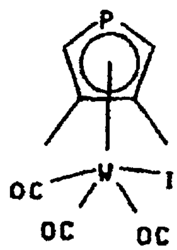

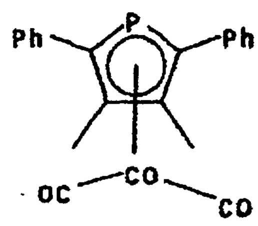

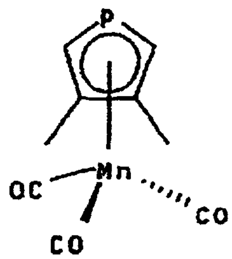

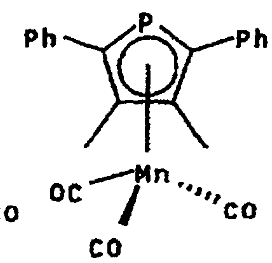

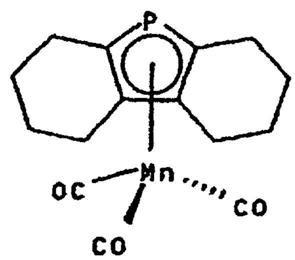

**3.** A complex of the formula (VI)

$$M'L'_{n'}(CO)_{m'} \qquad (VI)$$

where

M' is rhodium,

L' is a monodentate or polydentate $\eta^5$-phospholyl complex ligand capable of complex formation as claimed in claim 1 or 2 or a mixture thereof,

n' is at least 1,

m' is at least 1.

**4.** The use of a complex as claimed in claim 3 as catalyst in the hydroformylation of olefins.

**Revendications**

**1.** Procédé pour la fabrication d'aldéhydes par hydroformylation d'oléfines par $CO/H_2$ en présence de complexes de métaux de transition du VIIIème groupe auxiliaire de la classification périodique des éléments à des températures de 20 à 200°C et des pressions de la pression atmosphérique jusqu'à 700 bar, **caractérisé en ce qu'**on utilise comme ligands des catalyseurs comportant des complexes $\eta^5$-phosphyles capables de former des complexes, que le métal de transition utilisé comme $\eta^1$-métal st du rhodium et les complexes $\eta^5$-phosphyles sont choisis parmi les structures générales I et V

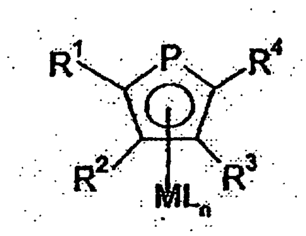

I

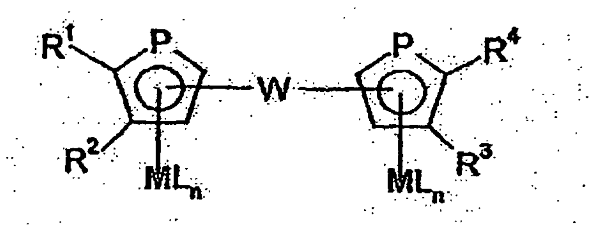

V

dans lesquelles $R^1$ à $R^4$ signifient, indépendamment l'un de l'autre, hydrogène, résidus $C_{1-12}$-alkyle, $C_{7-12}$-aralkyle, $C_{7-12}$-alkaryle ou $C_{6-12}$-aryle, où au moins un des substituants $R^1$ à $R^4$ peut présenter un groupement azote supplémentaire ou un groupement phosphore à trois liaisons capable de la coordination, de sorte qu'il se produit un ligand à deux ou plusieurs dents, ou représentent les résidus COO $\bar{M}^+$, $SO_3$ $\bar{M}^+$, $NR_3^+X$,OR, $NR_2$, COOR, SR, $(CHRCH_2O)_xR$, $(CH_2NR)_xR$ ou $(CH_2CH_2NR)_xR$, où R signifie des résidus identiques ou différents, choisis parmi hydrogène, résidus $C_{1-12}$-alkyle et $C_{6-12}$-aryle, $M^+$ un cation et X un anion et x les chiffres de 1 à 120, et où les résidus $R^1$ à $R^4$ peuvent être combinés à des cycles anellés, et dans lesquelles $ML_n$ représente un fragment de complexe métallique et W un pont sous forme d'une liaison covalente ou d'une chaîne de 1 à 10 atomes, qui peut être composant d'une combinaison cyclique ou aromatique, ou est une combinaison complexe, et où les unités de complexe $\eta^5$-phosphyle sont combinées au phosphore en 2 ou 3 emplacements, où les positions 2 et 3 non utilisées pour le pont peuvent être substituées par des résidus, comme décrit pour $R^1$ à $R^4$, et où un ou plusieurs des substituants $R^1$ à $R^4$ peuvent être un complexe $\eta^5$-phosphyle de formule I relié par un autre pont, où M est choisi parmi Zr, W, Mn, Fe, Ru, Co, et L est choisi parmi cyclopentadiènyle, CO, halogène et phosphane et des mélanges de ceux-ci.

2. Procédé selon la revendication 1, **caractérisé en ce que** les complexes $\eta^5$-phosphyles sont choisis parmi les structures suivantes, dans lesquelles Ph signifie phényle et Cy cyclohexyle.

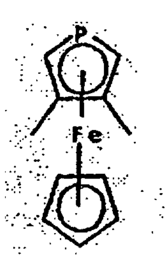

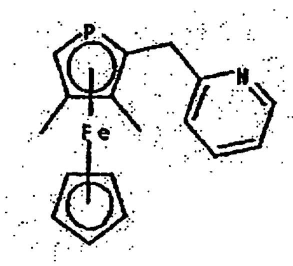

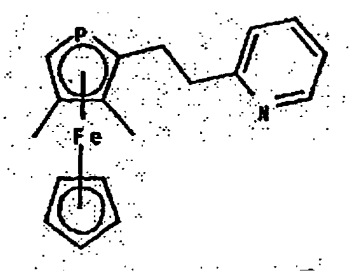

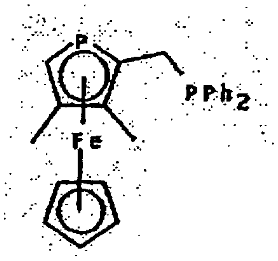
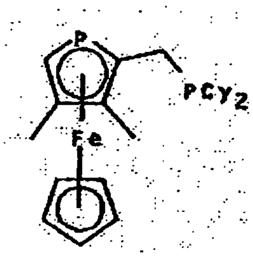
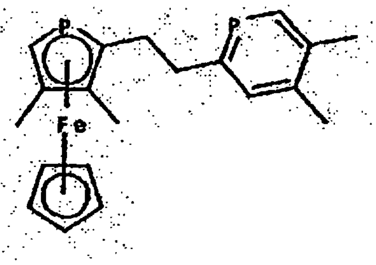

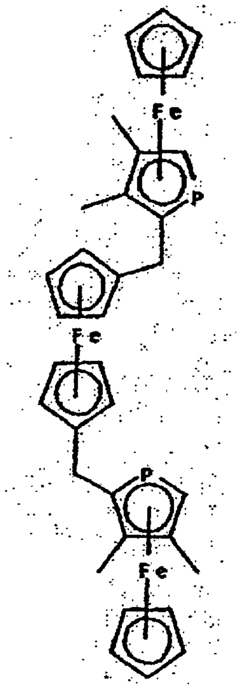
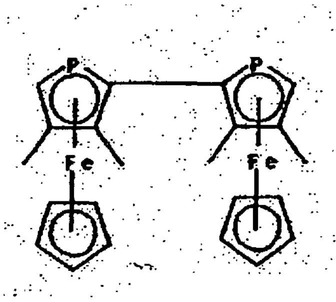
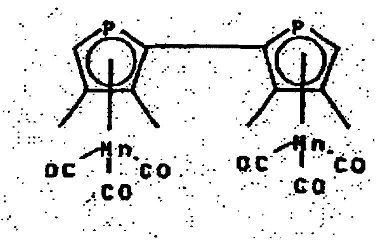

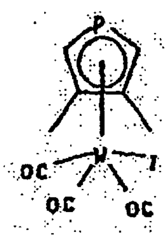
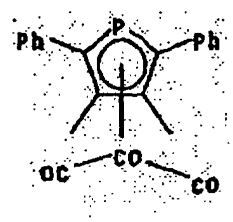

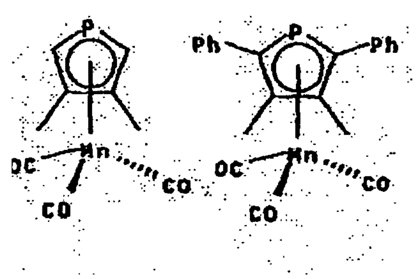
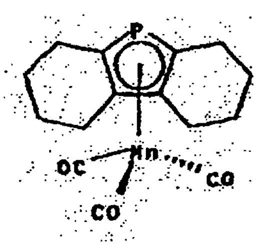

**3.** Complexe de formule générale (VI)

$$M'L'_{n'}(CO)_{m'} \qquad (VI)$$

ayant la signification

M' rhodium

L' ligand à une ou plusieurs dents, capable de la formation d'un complexe, constitué d'un complexe $\eta^5$-phosphyle, comme défini par la revendication 1 ou 2, ou un mélange de ligands de ce type,

n' au moins 1,

m' au moins 1.

**4.** Utilisation de complexes selon la revendication 3 comme catalyseur dans l'hydroformylation d'oléfines.